(19) Europäisches Patentamt / European Patent Office / Office européen des brevets

(11) **EP 2 242 423 B1**

(12) **EUROPEAN PATENT SPECIFICATION**

(45) Date of publication and mention
of the grant of the patent:
**13.04.2016  Bulletin 2016/15**

(51) Int Cl.:
***A61B 5/053*** (2006.01)

(21) Application number: **09710912.8**

(86) International application number:
**PCT/AU2009/000163**

(22) Date of filing: **12.02.2009**

(87) International publication number:
**WO 2009/100491 (20.08.2009 Gazette 2009/34)**

(54) **ANALYSING IMPEDANCE MEASUREMENTS**

ANALYSE VON IMPEDANZMESSUNGEN

ANALYSE DE MESURES D' IMPÉDANCE

(84) Designated Contracting States:
**AT BE BG CH CY CZ DE DK EE ES FI FR GB GR HR HU IE IS IT LI LT LU LV MC MK MT NL NO PL PT RO SE SI SK TR**

(30) Priority: **15.02.2008  US 29272**

(43) Date of publication of application:
**27.10.2010  Bulletin 2010/43**

(73) Proprietor: **Impedimed Limited**
**Pinkenba, QLD 4008 (AU)**

(72) Inventor: **GAW, Richelle Leanne**
**Greenslopes, Queensland 4120 (AU)**

(74) Representative: **Appleyard Lees**
**15 Clare Road**
**Halifax HX1 2HY (GB)**

(56) References cited:
**EP-A1- 1 177 760      EP-A1- 1 338 246**
**WO-A1-2005/122888    WO-A1-2007/002991**
**WO-A1-2007/002992    WO-A1-2007/002993**
**WO-A1-2007/041783    WO-A1-2007/045006**

US-A- 4 144 878        US-A1- 2005 177 062

- CORNISH BRUCE H ET AL: "A new technique for the quantification of peripheral edema with application in both unilateral and bilateral cases.", ANGIOLOGY 2002 JAN-FEB, vol. 53, no. 1, January 2002 (2002-01), pages 41-47, XP002695252, ISSN: 0003-3197
- SEO A ET AL: "Measuring lower leg swelling: Optimum frequency for impedance method", MEDICAL & BIOLOGICAL ENGINEERING & COMPUTING, SPRINGER, BERLIN, DE, vol. 39, no. 2, 1 March 2001 (2001-03-01), pages 185-189, XP019834171, ISSN: 1741-0444
- MCCULLAGH W A ET AL: "Bioelectrical impedance analysis measures the ejection fraction of the calf muscle pump", IFMBE PROCEEDINGS - 13TH INTERNATIONAL CONFERENCE ON ELECTRICAL BIOIMPEDANCE AND THE 8TH CONFERENCE ON ELECTRICAL IMPEDANCE TOMOGRAPHY 2007, ICEBI 2007 2007 SPRINGER VERLAG DEU, vol. 17 IFMBE, 2007, pages 616-619, XP002695280,
- FENECH M ET AL: "Extracellular and Intracellular Volume Variations During Postural Change Measured by Segmental and Wrist-Ankle Bioimpedance Spectroscopy", IEEE TRANSACTIONS ON BIOMEDICAL ENGINEERING, IEEE SERVICE CENTER, PISCATAWAY, NJ, USA, vol. 51, no. 1, 1 January 2004 (2004-01-01), pages 166-175, XP011104178, ISSN: 0018-9294, DOI: 10.1109/TBME.2003.820338

**Description**

**Background of the Invention**

The present invention relates to a method and apparatus for use in analysing impedance measurements, and in particular, to a method and apparatus for determining an indicator indicative of extracellular fluid levels using impedance measurements, the indicator being usable in identifying venous insufficiency, lymphoedema and/or oedema.

**Description of the Prior Art**

The reference in this specification to any prior publication (or information derived from it), or to any matter which is known, is not, and should not be taken as an acknowledgment or admission or any form of suggestion that the prior publication (or information derived from it) or known matter forms part of the common general knowledge in the field of endeavour to which this specification relates.

Venous insufficiency is a condition characterized by an inability for veins to adequately return blood to the heart. Normally, when a subject is in a standing position, the blood in the subject's leg veins is urged back towards the heart against gravity by a combination of mechanisms, such as muscular squeezing of the leg veins, and through the action of one-way valves in the veins. However, conditions can arise such as increased pressure within the veins, deep vein thrombosis (DVT), phlebitis, or the like, which lead to blood pooling in the legs.

Chronic venous disease (CVD) is common with a 3-7% prevalence, resulting in an economic cost US$1 billion per annum.

Typical detection methods for venous insufficiency involve examining for physical symptoms such as swelling in the leg or ankle, tightness in the calves, leg tiredness, pain while walking, or the like. Venous insufficiency may also be associated with varicose veins.

Other techniques for assessing venous insufficiency include measuring the ambulatory venous pressure, which is achieved by inserting a needle into the vein on the dorsum of the foot. Whilst this is regarded as the gold standard of haemodynamic investigation, this is invasive, and it is therefore desirable to find alternative non-invasive techniques. Two such methods are air plethysmography (APG) and strain gauge plethysmography (SPG).

SPG involves placing mercury strain gauges in a silastic band around the calf muscle which are calibrated to read percentage leg volume changes, as described for example in Nicolaides AN (2000) "Investigation of Chronic Venous Insufficiency: A Consensus Statement" Circulation 102:126-163. These measurements are typically performed during exercise regimens to allow venous refilling time and the ejection volume to be assessed. APG uses an air bladder which surrounds the leg from the knee to the ankle. The bladder is inflated to a known pressure, with volume changes in the calf muscle being determined based on changes in pressure on the bladder during a sequence of postural changes.

However, these techniques are only of limited accuracy, and can require extensive calibration and exercise, to allow useable measurement to be obtained.

Lymphoedema is a condition characterised by excess protein and oedema in the tissues as a result of reduced lymphatic transport capacity and/or reduced tissue proteolytic capacity in the presence of a normal lymphatic load. Acquired, or secondary lymphoedema, is caused by damaged or blocked lymphatic vessels. The commonest inciting events are surgery and/or radiotherapy. However, onset of lymphoedema is unpredictable and may develop within days of its cause or at any time during a period of many years after that cause.

One existing technique for determining biological parameters relating to a subject, such as fluid levels, involves the use of bioelectrical impedance. This involves measuring the electrical impedance of a subject's body using a series of electrodes placed on the skin surface. Changes in electrical impedance at the body's surface are used to determine parameters, such as changes in fluid levels, associated with the cardiac cycle or oedema.

US2006/0111652 describes methods for enhancing blood and lymph flow in the extremities of a human. As part of this method, impedance measurements are used to assess segmental blood flows within the limbs.

US2005/0177062 describes a system for measuring the volume, composition and the movement of electroconductive body fluids, based on the electrical impedance of the body or a body segment. This is used primarily for electromechanocardiography (ELMEC) or impedance cardiography (IKG) measurements for determining hemodynamic parameters.

WO00/79255 describes a method of detection of oedema by measuring bioelectrical impedance at two different anatomical regions in the same subject at a single low frequency alternating current. The two measurements are analysed to obtain an indication of the presence of tissue oedema by comparing with data obtained from a normal population.

"Extracellular and Intracellular Volume Variations During Postural Change Measured by Segmental and Wrist-ankle Bioimpedance Spectroscopy" by Marianne Fenech and Michel Y. Jeffrin IEEE TRANSACTIONS ON BIOMEDICAL ENGINEERING vol. 51, 1 January 2004 (2004-01-01), pages 166-175 describes measuring extracellular and intracellular volumes using segmental and write-ankle Bioimpedance Spectroscopy (BIS). A number of protocols are described including sitting and laying supine which shows that when switching from sitting to supine, the mean extracellular volumes increase with corresponding increases in intracellular volumes being smaller.

**Summary of the Present Invention**

The present invention seeks to ameliorate one or more problems of the problems associated with the prior art.
In a first broad form the present invention seeks to provide a method for use in analysing impedance measurements performed on a subject, the method including, in a processing system:

a) determining first and second impedance values indicative of the impedance of at least one leg segment of the subject;
b) determining an indicator using the first and second impedance values, the indicator being indicative of extracellular fluid levels in the at least leg segment and being used in the assessment of venous insufficiency; to determine the indicator by:

i) determining a first indicator value with the subject in a first orientation using the first impedance value;
ii) after positioning the subject in a second orientation for a predetermined time period, determining a second indicator value with the subject in the first orientation using the second impedance value; and,
iii) determining the indicator using a difference between the first and second fluid indicator values, characterised in that the processing system is operable to:

(a) monitor the difference;
(b) determine the time taken for the difference to fall below a reference; and,
(c) provide an indication of the time taken to allow determination of a presence, absence, or degree of venous insufficiency.

Typically the method includes, in a processing system:

a) comparing the indicator to a reference; and,
b) providing an indication of the results of the comparison to allow determination of a presence, absence or degree of venous insufficiency.

Typically the method includes, in a processing system, determining an index using the at least one impedance value, the index being indicative of a ratio of extracellular to intracellular fluid levels in the at least leg segment, the index being used in the assessment of venous insufficiency.
Typically the method includes, in the processing system:

a) comparing the index to a reference; and,
b) providing an indication of the results of the comparison to allow determination of a presence, absence or degree of venous insufficiency.

Typically the method includes, diagnosing the presence of venous insufficiency if:

a) an indicator is less than first reference; and,
b) an index is greater than a second reference.

Typically the method includes, in a processing system:

a) determining a first index value with the subject in a first orientation;
b) determining a second index value with the subject in a second orientation; and,
c) determining an index change based on a difference between the first and second fluid index values, the index change being used in the assessment of venous insufficiency.

Typically the method includes, in the processing system:

a) determining a first indicator value with the subject in a first orientation;
b) after positioning the subject in a second orientation for a predetermined time period, determining a second indicator value with the subject in the first orientation; and,

c) determining a difference between the first and second fluid indicator values, the difference being used in the assessment of venous insufficiency.

Typically the method includes, in a processing system:

a) determining a pre-treatment indicator value prior to treatment of the subject;
b) determining a post-treatment indicator value following treatment of the subject for venous insufficiency; and,
c) determining an indicator change based on a difference between the pre-treatment and post-treatment indicator values, the indicator change being used in the assessment of venous insufficiency.

Typically the method includes, diagnosing the presence of venous insufficiency if the indicator change is greater than a reference.

Typically the reference includes using a reference that is at least one of:

a) an indicator or index determined for another limb of the subject;
b) a reference determined from a sample population; and,
c) a previous indicator or index determined for the subject.

Typically the method includes, in the processing system, displaying at least one of:

a) an indicator;
b) an index ratio;
c) an index;
d) an indicator change;
e) an index change;
f) one or more impedance parameter values; and,
g) results of a comparison.

Typically the first impedance is measured at a measurement frequency of at least one of:

a) less than 100 kHz;
b) less than 50 kHz; and,
c) less than 10 kHz.

Typically the method includes, in the processing system, using the at least one impedance measurement as an estimate of a resistance of the subject at a zero measurement frequency.

Typically the method includes measuring at least one second impedance value, the at least one second impedance value being measured at a measurement frequency of at least one of:

a) greater than 200 kHz;
b) greater than 500 kHz; and,
c) greater than 1000 kHz.

Typically the method includes, in the processing system, using the at least one second impedance measurement as an estimate of a resistance of the subject at an infinite measurement frequency.

Typically the method includes, in the processing system:

a) determining a plurality of impedance values; and,
b) determining at least one impedance parameter value from the plurality of impedance values.

Typically the impedance parameter values include at least one of:

$R_0$ which is the resistance at zero frequency;
$R_\infty$ which is the resistance at infinite frequency; and,
$Z_c$ which is the resistance at a characteristic frequency.

Typically the method includes, in the processing system:

a) determining values for impedance parameters $R_0$ and $R_\infty$ from the measured impedance values; and,

b) calculating the index ($I$) using the equation:

$$I \doteq \frac{R_\infty}{R_0 - R_\infty}$$

Typically the method includes, in the processing system, determining the parameter values using the equation:

$$Z = R_\infty + \frac{R_0 - R_\infty}{1 + (j\omega\tau)^{(1-\alpha)}}$$

where:

Z is the measured impedance at angular frequency $\omega$,
$\tau$ is a time constant, and
$\alpha$ has a value between 0 and 1.

Typically the method includes, in the computer system, causing the impedance measurements to be performed.

Typically the method includes, in the computer system:

a) causing one or more electrical signals to be applied to the subject using a first set of electrodes;
b) measuring electrical signals across a second set of electrodes applied to the subject in response to the applied one or more signals; and,
c) determining from the applied signals and the measured signals at least one measured impedance value.

In a second broad form the present invention seeks to provide apparatus for use in analysing impedance measurements performed on a subject, the apparatus including a processing system for:

a) determining first and second impedance values, indicative of the impedance of at least leg segment of the subject;
b) determining an indicator using the first and second impedance values, the indicator being indicative of extracellular fluid levels in the at least leg segment and being used in the assessment of venous insufficiency; and wherein the method further includes, in the processing system determining the indicator by :

i) determining a first indicator value with the subject in a first orientation using the first impedance value,
ii) after positioning the subject in a second orientation for a predetermined time period, determining a second indicator value with the subject in the first orientation using the second impedance value; and,
iii) determining the indicator using the difference between the first and second fluid indicator values, characterised in that the method further comprises, in the processing system:

(a) monitor the difference;
(b) determine the time taken for the difference to fall below a reference; and,
(c) provide an indication of the time taken to allow determination of a presence, absence or degree of venous insufficiency.

Typically the apparatus includes a processing system for:

a) causing one or more electrical signals to be applied to the subject using a first set of electrodes;
b) measuring electrical signals across a second set of electrodes applied to the subject in response to the applied one or more signals; and,
c) determining from the applied signals and the measured signals at least one measured impedance value.

Typically the apparatus includes:

a) a signal generator for generating electrical signals; and,
b) a sensor for sensing electrical signals.

It will be appreciated that the broad forms of the invention may be used individually or in combination, and may be used for assessing venous insufficiency as well as diagnosing the presence, absence or degree of a range of conditions in addition to and including oedema, lymphodema, body composition, or the like.

**Brief Description of the Drawings**

An example of the present invention will now be described with reference to the accompanying drawings, in which: -

Figure 1 is a schematic diagram of a first example of impedance measuring apparatus;
Figure 2 is a flowchart of an example of a process for use in analysing impedance measurements;
Figure 3 is a schematic diagram of a second example of impedance measuring apparatus;
Figure 4 is a schematic diagram of an example of a computer system;
Figure 5 is a flowchart of an example of a process for performing impedance measurements;
Figure 6A is a schematic of an example of a theoretical equivalent circuit for biological tissue;
Figure 6B is an example of a locus of impedance known as a Wessel plot;
Figure 7 is a flowchart of a first comparative example of a process for analysing impedance measurements to allow assessment of venous insufficiency;
Figure 8 is a flowchart of a second comparative example of a process for analysing impedance measurements to allow assessment of venous insufficiency;
Figure 9 is a flowchart of a third comparative example of a process for analysing impedance measurements to allow assessment of venous insufficiency;
Figure 10 is a flowchart of a first specific example of a process for analysing impedance measurements to allow assessment of venous insufficiency; and,
Figure 11 is a flowchart of a second specific example of a process for analysing impedance measurements to allow assessment of venous insufficiency.

**Detailed Description of the Drawings**

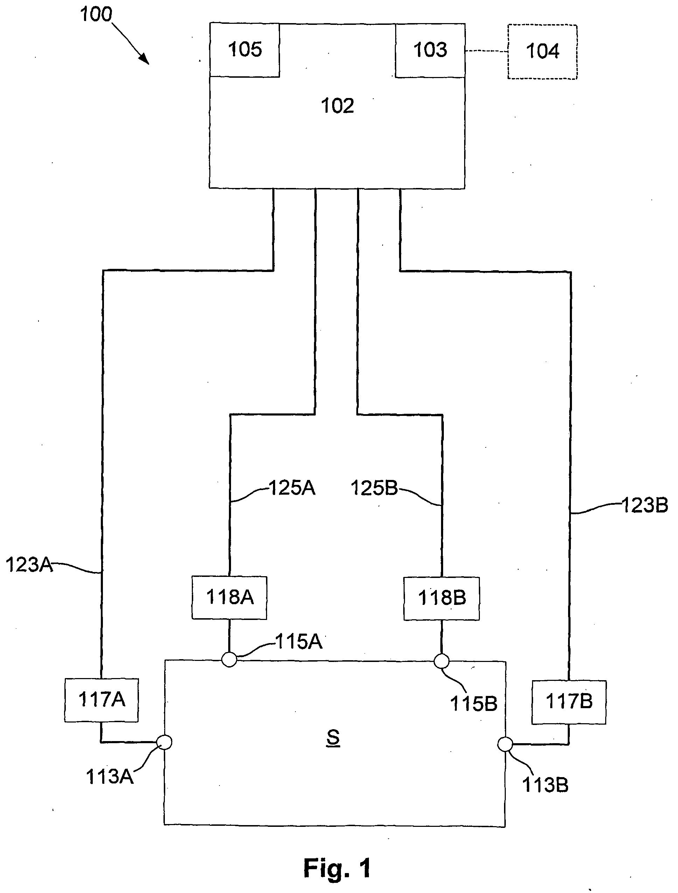

An example of apparatus suitable for performing an analysis of a subject's bioelectric impedance will now be described with reference to Figure 1.

As shown the apparatus includes a measuring device 100 including a processing system 102, connected to one or more signal generators 117A, 117B, via respective first leads 123A, 123B, and to one or more sensors 118A, 118B, via respective second leads 125A, 125B. The connection may be via a switching device, such as a multiplexer, although this is not essential.

In use, the signal generators 117A, 117B are coupled to two first electrodes 113A, 113B, which therefore act as drive electrodes to allow signals to be applied to the subject S, whilst the one or more sensors 118A, 118B are coupled to the second electrodes 115A, 115B, which therefore act as sense electrodes, to allow signals induced across the subject S to be sensed.

The signal generators 117A, 117B and the sensors 118A, 118B may be provided at any position between the processing system 102 and the electrodes 113A, 113B, 115A, 115B, and may therefore be integrated into the measuring device 100.

However, in one example, the signal generators 117A, 117B and the sensors 118A, 118B are integrated into an electrode system, or another unit provided near the subject S, with the leads 123A, 123B, 125A, 125B connecting the signal generators 117A, 117B and the sensors 118A, 118B to the processing system 102. By performing this, the length of any connections between the signal generators 117A, 117B and the sensors 118A, 118B, and the corresponding electrodes 113A, 113B, 115A, 115B can be reduced. This minimises any parasitic capacitances between the connections, the connections and the subject, and the connections and any surrounding articles, such as a bed on which the subject is provided, thereby reducing measurement errors.

The above described system can be described as a two channel device, with each channel being designated by the suffixes A, B respectively. The use of a two channel device is for the purpose of example only, and any number of channels may be provided, as required.

An optional external interface 103 can be used to couple the measuring device 100, via wired, wireless or network connections, to one or more peripheral devices 104, such as an external database or computer system, barcode scanner, or the like. The processing system 102 will also typically include an I/O device 105, which may be of any suitable form such as a touch screen, a keypad and display, or the like.

In use, the processing system 102 is adapted to generate control signals, which cause the signal generators 117A, 117B to generate one or more alternating signals, such as voltage or current signals of an appropriate waveform, which can be applied to a subject S, via the first electrodes 113A, 113B. The sensors 118A, 118B then determine the voltage across or current through the subject S, using the second electrodes 115A, 115B and transfer appropriate signals to the processing system 102.

Accordingly, it will be appreciated that the processing system 102 may be any form of processing system which is suitable for generating appropriate control signals and at least partially interpreting the measured signals to thereby determine the subject's bioelectrical impedance, and optionally determine other information such indicators of the presence, absence or degree of venous insufficiency, other conditions, or the like.

The processing system 102 may therefore be a suitably programmed computer system, such as a laptop, desktop, PDA, smart phone or the like. Alternatively the processing system 102 may be formed from specialised hardware, such as an FPGA (field programmable gate array), or a combination of a programmed computer system and specialised hardware, or the like, as will be described in more detail below.

In use, the first electrodes 113A, 113B are positioned on the subject to allow one or more signals to be injected into the subject S. The location of the first electrodes will depend on the segment of the subject S under study. Thus, for example, the first electrodes 113A, 113B can be placed on the thoracic and neck region of the subject S to allow the impedance of the chest cavity to be determined for use in cardiac function analysis. Alternatively, positioning electrodes on the wrist and ankles of a subject allows the impedance of limbs and/or the entire body to be determined, for use in oedema analysis, assessment of venous insufficiency, or the like.

Once the electrodes are positioned, one or more alternating signals are applied to the subject S, via the first electrodes 113A, 113B. The nature of the alternating signal will vary depending on the nature of the measuring device and the subsequent analysis being performed.

For example, the system can use Bioimpedance Analysis (BIA) in which a single low frequency signal is injected into the subject S, with the measured impedance being used directly in the determination of biological parameters, such as extracellular fluid levels, which can be indicative of oedema, and hence of venous insufficiency.

In one example, the applied signal has a relatively low frequency, such as below 100 kHz, more typically below 50 kHz and more preferably below 10 kHz. In this instance, such low frequency signals can be used as an estimate of the impedance at zero applied frequency, commonly referred to as the impedance parameter value $R_0$, which is in turn indicative of extracellular fluid levels.

Alternatively, the applied signal can have a relatively high frequency, such as above 200 kHz, and more typically above 500 kHz, or 1000 kHz. In this instance, such high frequency signals can be used as an estimate of the impedance at infinite applied frequency, commonly referred to as the impedance parameter value $R_\infty$, which is in turn indicative of a combination of the extracellular and intracellular fluid levels, as will be described in more detail below.

In contrast Bioimpedance Spectroscopy (BIS) devices perform impedance measurements at multiple frequencies over a selected frequency range. Whilst any range of frequencies may be used, typically frequencies range from very low frequencies (4 kHz) to higher frequencies (15000 kHz). Similarly, whilst any number of measurements may be made, in one example the system can use 256 or more different frequencies within this range, to allow multiple impedance measurements to be made within this range.

When impedance measurements are made at multiple frequencies, these can be used to derive one or more impedance parameter values, such as values of $R_0$, $Z_c$, $R_\infty$, which correspond to the impedance at zero, characteristic and infinite frequencies. These can in turn be used to determine information regarding both intracellular and extracellular fluid levels, as will be described in more detail below.

A further alternative is for the system to use Multiple Frequency Bioimpedance Analysis (MFBIA) in which multiple signals, each having a respective frequency are injected into the subject S, with the measured impedances being used in the assessment of fluid levels. In one example, four frequencies can be used, with the resulting impedance measurements at each frequency being used to derive impedance parameter values, for example by fitting the measured impedance values to a Cole model, as will be described in more detail below. Alternatively, the impedance measurements at each frequency may be used individually or in combination.

Thus, the measuring device 100 may either apply an alternating signal at a single frequency, at a plurality of frequencies simultaneously, or a number of alternating signals at different frequencies sequentially, depending on the preferred implementation. The frequency or frequency range of the applied signals may also depend on the analysis being performed.

In one example, the applied signal is generated by a voltage generator, which applies an alternating voltage to the subject S, although alternatively current signals may be applied.

In one example, the voltage source is typically symmetrically and/or differentially arranged, with each of the signal generators 117A, 117B being independently controllable, to allow the potential across the subject to be varied. This can be performed to reduce the effects of any imbalance, which occurs when the voltages sensed at the electrodes are unsymmetrical (a situation referred to as an "imbalance"). In this instance, any difference in the magnitude of signals within the leads can lead to differing effects due to noise and interference.

Whilst applying the voltage symmetrically, can reduce the effect, this is not always effective if the electrode impedances for the two drive electrodes 113A, 113B are unmatched, which is typical in a practical environment. However, by adjusting the differential drive voltages applied to each of the drive electrodes 113A, 113B, this compensates for the different electrode impedances, and restores the desired symmetry of the voltage at the sense electrodes 115A, 115B. This can be achieved by measuring the voltages at the sense electrodes, and then adjusting the magnitude and/or phase of the applied signal to thereby balance the magnitude of the sensed voltages. This process is referred to herein as *balancing* and in one example is performed by minimizing the magnitude of any common mode signal.

A potential difference and/or current is measured between the second electrodes 115A, 115B. In one example, the voltage is measured differentially, meaning that each sensor 118A, 118B is used to measure the potential at each second electrode 115A, 115B and therefore need only measure half of the potential as compared to a single ended system.

The acquired signal and the measured signal will be a superposition of potentials generated by the human body, such as the ECG (electrocardiogram), potentials generated by the applied signal, and other signals caused by environmental electromagnetic interference. Accordingly, filtering or other suitable analysis may be employed to remove unwanted components.

The acquired signal is typically demodulated to obtain the impedance of the system at the applied frequencies. One suitable method for demodulation of superposed frequencies is to use a Fast Fourier Transform (FFT) algorithm to transform the time domain data to the frequency domain. This is typically used when the applied current signal is a superposition of applied frequencies. Another technique not requiring windowing of the measured signal is a sliding window FFT.

In the event that the applied current signals are formed from a sweep of different frequencies, then it is more typical to use a signal processing technique such as correlating the signal. This can be achieved by multiplying the measured signal with a reference sine wave and cosine wave derived from the signal generator, or with measured sine and cosine waves, and integrating over a whole number of cycles. This process, known variously as quadrature demodulation or synchronous detection, rejects all uncorrelated or asynchronous signals and significantly reduces random noise.

Other suitable digital and analogue demodulation techniques will be known to persons skilled in the field.

In the case of BIS, impedance or admittance measurements can be determined from the signals at each frequency using the recorded voltage across and current flow through the subject. The demodulation algorithm can then produce an amplitude and phase signal at each frequency. This can then be used to derive one or more impedance parameter values, if required.

As part of the above described process, the position of the second electrodes may be measured and recorded. Similarly, other parameters relating to the subject (subject parameters) may be recorded, such as the height, weight, age, sex, health status, any interventions and the date and time on which they occurred. Other information, such as current medication, may also be recorded. This can then be used in performing further analysis of the impedance measurements, so as to allow determination of the presence, absence or degree of venous insufficiency and/or oedema, to assess body composition, or the like.

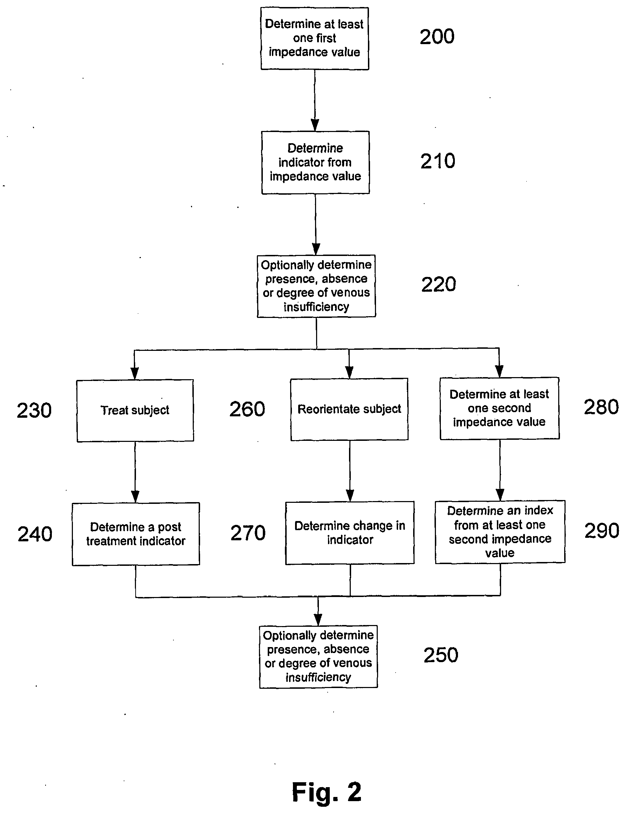

An example of the process of analysing impedance measurements operation of the apparatus of Figure 1 to perform this will now be described with reference to Figure 2.

At step 200, at least one first impedance value indicative of the impedance of at least one segment of the subject's leg is determined. This may be achieved by having the signal generators 117A, 117B, apply at least one first signal to the subject S, via the first electrodes 113A, 113B, with voltage signals being measured across the subject S by the sensors 118A, 118B, via the second electrodes 115A, 115B. An indication of the current flow through and voltage across the subject is provided to the processing system 102, allowing the impedance, or an impedance parameter value to be determined.

At step 210, an indicator is determined using the first impedance. The indicator is typically indicative of the extracellular fluid levels within the subject. Accordingly, in one example, the impedance measurement is performed at a single low frequency, such as below 100 kHz, and in one example, at 5 kHz, allowing the indicator to be based on the measured value directly. Alternatively, multiple measurements may be performed at multiple frequencies, with the indicator being based on an appropriate impedance parameter value derived therefrom, such as the impedance at zero applied frequency $R_0$, as will be described in more detail below.

Optionally, at step 220, the indicator can be used in the assessment of venous insufficiency, or other conditions, such as oedema or lymphoedema. In this regard, high extracellular fluid levels in the leg segment are indicative of oedema in the leg, which is in turn an indicator that venous insufficiency may be present. In one example, the indicator can be compared to a reference, such as an oedema reference, to allow the presence, absence or degree of oedema to be determined, as will be described in more detail below.

As the determination of the presence of oedema alone may not be conclusive as to the presence, absence or degree of venous insufficiency, or lymphoedema, additional steps may optionally be performed.

In one example, at step 230, the subject can be treated for venous insufficiency. This can be performed in any one of a number of manners, such as by performing ablation, or the like. Following this, at step 240, the impedance measurement described above is repeated to allow a post-treatment indicator to be determined. Any difference between the pre-treatment indicator determined prior to treatment, and the post-treatment indicator, can be used to determine if there has been a reduction in the extra-cellular fluid levels. Any reduction in fluid levels indicates that the treatment has been at least partially successful, thereby allowing the presence of venous insufficiency to be confirmed at step 250.

In this example, both measurements are typically made with the subject in a standing or equivalent position, such as leaning or sitting with their leg hanging in a substantially vertical position, to thereby enhance the impact of blood pooling caused by venous insufficiency on extracellular fluid levels. For the purpose of the remaining description, the term standing will be understood to encompass any position that maximises or enhances pooling of blood in the subject's leg.

In another example, at step 260, the subject is reorientated from an orientation used to determine the first indicator, allowing a change in indicator between different orientations to be determined at step 270.

Thus, the first indicator can be determined with the subject provided in a first orientation, to allow a baseline reading to be established. In one example, this is performed with the subject in an orientation designed to reduce or minimise blood pooling, such as with the subject in a supine position, and optionally with their leg elevated to a height of up to 20 cm above the level of their heart. For the purpose of the remaining description, the term supine will be understood to encompass any position that minimises pooling of blood in the subject's leg.

The subject then stands, leans or sits with their leg hanging in a substantially vertical position, allowing a second indicator to be determined, with a change in indicator values being indicative of the change of extracellular fluid levels within the leg, which in turn can be used in venous insufficiency assessment.

Alternatively, the subject can be allowed to stand to allow pooling of blood. Following this, the subject is returned to the supine position allowing the indicator value to be monitored. The time taken for this return to the baseline, or to within a range of the threshold, can then be used in venous insufficiency assessment.

In another example, at step 280, second impedance measurements are performed to allow an index to be determined at step 290. The index is typically indicative of a ratio between intracellular and extracellular fluid levels in the leg, or a leg segment, which can in turn be used to assess the presence of lymphoedema. Being able to distinguish between oedema and lymphoedema can assist in assessing venous insufficiency.

In the above described examples, the measurements are performed on the subject's leg as this maximises the effect of any blood pooling, thereby maximising the effectiveness of the measurement procedure to determine indicators that can be used in identifying venous insufficiency. However, if the technique is being used for other identifying conditions such as lymphoedema, then the process can be applied to other body segments, such as arms, as will be described in more detail below.

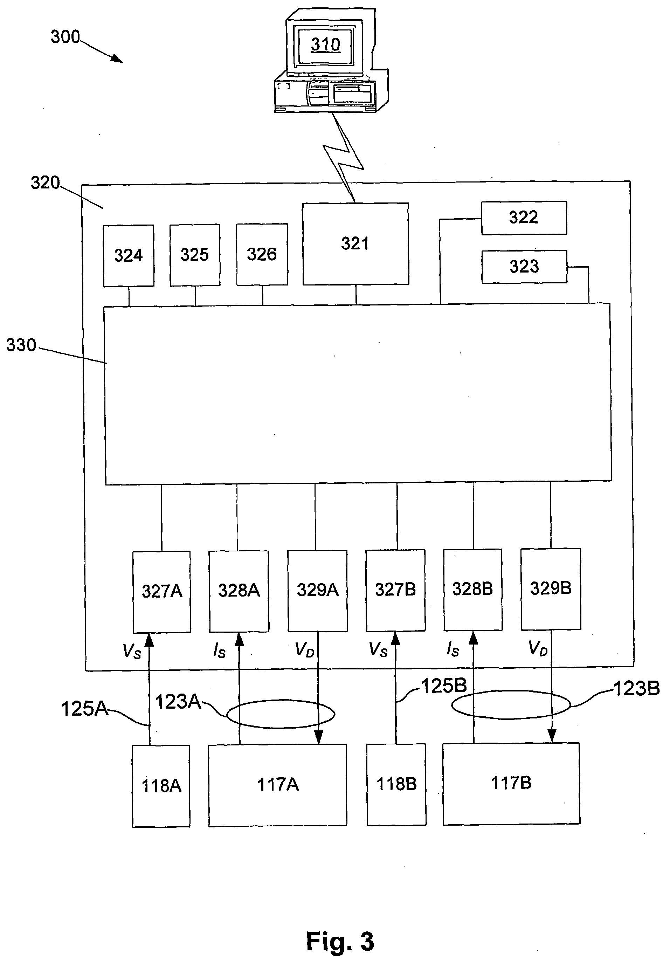

A specific example of the apparatus will now be described in more detail with respect to Figure 3.

In this example, the measuring system 300 includes a computer system 310 and a separate measuring device 320. The measuring device 320 includes a processing system 330 coupled to an interface 321 for allowing wired or wireless communication with the computer system 310. The processing system 330 may also be optionally coupled to one or more stores, such as different types of memory, as shown at 322, 323, 324, 325, 326.

In one example, the interface is a Bluetooth stack, although any suitable interface may be used. The memories can include a boot memory 322, for storing information required by a boot-up process, and a programmable serial number memory 323, that allows a device serial number to be programmed. The memory may also include a ROM (Read Only Memory) 324, flash memory 325 and EPROM (Electronically Programmable ROM) 326, for use during operation. These may be used for example to store software instructions and to store data during processing, as will be appreciated by persons skilled in the art.

A number of analogue to digital converters (ADCs) 327A, 327B, 328A, 328B and digital to analogue converters (DACs) 329A, 329B are provided for coupling the processing system 330 to the sensors 118A, 118B and the signal generators 117A, 117B, as will be described in more detail below.

A controller, such as a microprocessor, microcontroller or programmable logic device, may also be provided to control activation of the processing system 330, although more typically this is performed by software instructions executed by the processing system 330.

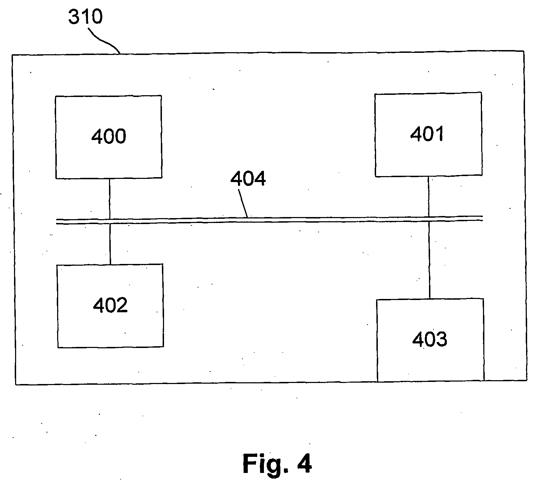

An example of the computer system 310 is shown in Figure 4. In this example, the computer system 310 includes a processor 400, a memory 401, an input/output device 402 such as a keyboard and display, and an external interface 403 coupled together via a bus 404, as shown. The external interface 403 can be used to allow the computer system to communicate with the measuring device 320, via wired or wireless connections, as required, and accordingly, this may be in the form of a network interface card, Bluetooth stack, or the like. In use, the computer system 310 can be used to control the operation of the measuring device 320, although this may alternatively be achieved by a separate interface provided on the measuring device 300. Additionally, the computer system 310 can be used to allow at least part of the analysis of the impedance measurements to be performed.

Accordingly, the computer system 310 may be formed from any suitable processing system, such as a suitably programmed PC, Internet terminal, lap-top, hand-held PC, smart phone, PDA, server, or the like, implementing appropriate applications software to allow required tasks to be performed.

In contrast, the processing system 330 typically performs specific processing tasks, to thereby reduce processing requirements on the computer system 310. Thus, the processing system typically executes instructions to allow control signals to be generated for controlling the signal generators 117A, 117B, as well as the processing to determine instantaneous impedance values.

In one example, the processing system 330 is formed from custom hardware, or the like, such as a Field Programmable Gate Array (FPGA), although any suitable processing module, such as a magnetologic module, may be used.

In one example, the processing system 330 includes programmable hardware, the operation of which is controlled using instructions in the form of embedded software instructions. The use of programmable hardware allows different signals to be applied to the subject S, and allows different analysis to be performed by the measuring device 320. Thus, for example, different embedded software would be utilised if the signal is to be used to analyse the impedance at a number of frequencies simultaneously as compared to the use of signals applied at different frequencies sequentially.

The embedded software instructions used can be downloaded from the computer system 310. Alternatively, the instructions can be stored in memory such as the flash memory 325 allowing the instructions used to be selected using either an input device provided on the measuring device 320, or by using the computer system 310. As a result, the computer system 310 can be used to control the instructions, such as the embedded software, implemented by the processing system 330, which in turn alters the operation of the processing system 330.

Additionally, the computer system 310 can operate to analyse impedance determined by the processing system 330, to allow biological parameters to be determined.

Whilst an alternative arrangement with a single processing system may be used, the division of processing between the computer system 310 and the processing system 330 can provide some benefits.

Firstly, the use of the processing system 330 more easily allows the custom hardware configuration to be adapted through the use of appropriate embedded software. This in turn allows a single measuring device to be used to perform a range of different types of analysis.

Secondly, the use of a custom configured processing system 330 reduces the processing requirements on the computer system 310. This in turn allows the computer system 310 to be implemented using relatively straightforward hardware, whilst still allowing the measuring device to perform sufficient analysis to provide interpretation of the impedance. This can include for example generating a "Wessel" plot, using the impedance values to determine parameters relating to cardiac function, as well as determining the presence or absence of lymphoedema.

Thirdly, this allows the measuring device 320 to be updated. Thus for example, if an improved analysis algorithm is created, or an improved current sequence determined for a specific impedance measurement type, the measuring device can be updated by downloading new embedded software via flash memory 325 or the external interface 321.

In use, the processing system 330 generates digital control signals, which are converted to analogue voltage drive signals $V_D$ by the DACs 329, and transferred to the signal generators 117. Analogue signals representing the current of the drive signal $I_D$ applied to the subject and the subject voltage $V_S$ measured at the second electrodes 115A, 115B are received from the signal generators 117 and the sensors 118 and are digitised by the ADCs 327, 328. The digital signals can then be returned to the processing system 330 for preliminary analysis.

In this example, a respective set of ADCs 327, 328, and DACs 329 are used for each of two channels, as designated by the reference numeral suffixes A, B respectively. This allows each of the signal generators 117A, 117B to be controlled independently and for the sensors 118A, 118B to be used to detect signals from the electrodes 115A, 115B respectively. This therefore represents a two channel device, each channel being designated by the reference numerals A, B.

In practice, any number of suitable channels may be used, depending on the preferred implementation. Thus, for example, it may be desirable to use a four channel arrangement, in which four drive and four sense electrodes are provided, with a respective sense electrode and drive electrode pair being coupled to each limb. In this instance, it will be appreciated that an arrangement of eight ADCs 327, 328, and four DACs 329 could be used, so each channel has respective ADCs 327, 328, and DACs 329. Alternatively, other arrangements may be used, such as through the inclusion of a multiplexing system for selectively coupling a two-channel arrangement of ADCs 327, 328, and DACs 329 to a four channel electrode arrangement, as will be appreciated by persons skilled in the art.

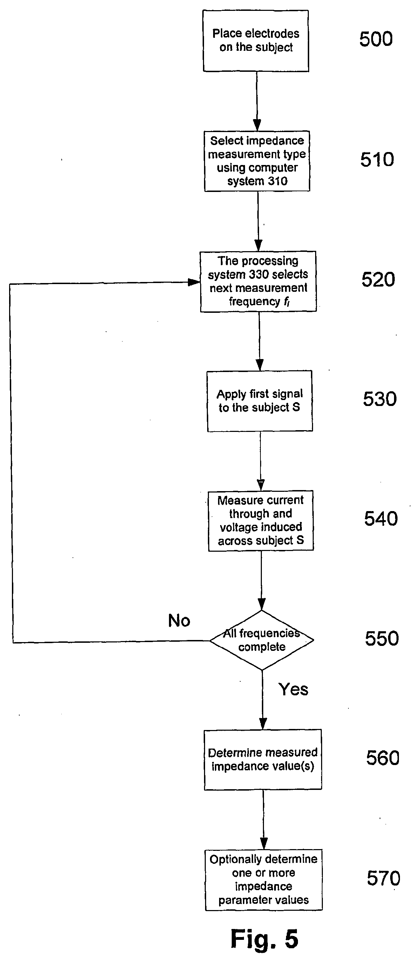

An example of the process for performing impedance measurements will now be described with reference to Figure 5.

At step 500, the electrodes are positioned on the subject as required. The general arrangement to allow impedance of a leg to be determined is to provide drive electrodes 113A, 113B on the hand at the base of the knuckles and on the feet at the base of the toes, on the side of the body being measured. Sense electrode 115A are also positioned at the front of the ankle on the leg being measured, with the sense electrode 115B being positioned anywhere on the contra-lateral leg.

It will be appreciated that this configuration uses the theory of equal potentials, allowing the electrode positions to provide reproducible results for impedance measurements. This is advantageous as it greatly reduces the variations in measurements caused by poor placement of the electrodes by the operator.

Alternatively however other arrangements can be used. Thus for example, the sense electrodes can be provided anywhere on the leg of interest, allowing the impedance measurements to be made along the entire leg, or for a part of the leg (generally referred to as a leg segment), such as a calf segment, or the like.

At step 510, an impedance measurement type is selected using the computer system 310, allowing the processing system to determine an impedance measurement protocol, and configure the processing system 330 accordingly. This is typically achieved by configuring firmware or software instructions within the processing system 330, as described above.

At step 520, the processing system 300 selects a next measurement frequency $f_i$, and causes the signal generators 117A, 117B to apply a first signal to the subject at the selected frequency at step 530. At step 540, the signal generators 117A, 117B and sensors 118A, 118B provide an indication of the current through and the voltage across the leg segment to the processing system 330.

At step 550, the processing system 330 determines if all frequencies are complete, and if not returns to step 520 to select the next measurement frequency. At step 560, one or more measured impedance values are determined, by the computer system 310, the processing system 330, or a combination thereof, using the techniques described above. One or more impedance parameter values may optionally be derived at step 570.

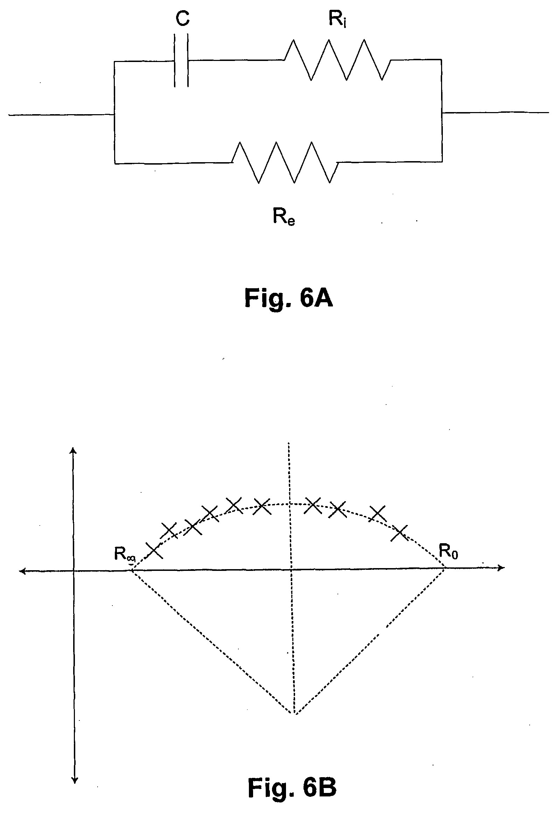

In this regard, Figure 6A is an example of an equivalent circuit that effectively models the electrical behaviour of biological tissue. The equivalent circuit has two branches that represent current flow through extracellular fluid and intracellular fluid, respectively. The extracellular fluid component of biological impedance is represented by an extracellular resistance $R_e$, whilst the intracellular fluid component is represented by an intracellular resistance $R_i$ and a capacitance C representative of the cell membranes.

The relative magnitudes of the extracellular and intracellular components of impedance of an alternating current (AC) are frequency dependent. At zero frequency the capacitor acts as a perfect insulator and all current flows through the extracellular fluid, hence the resistance at zero frequency, $R_0$, equals the extracellular resistance $R_e$. At infinite frequency the capacitor acts as a perfect conductor and the current passes through the parallel resistive combination. The resistance at infinite frequency $R_\infty$ is given by:

$$R_\infty = \frac{R_e R_i}{R_e + R_i} \tag{1}$$

Accordingly, the impedance of the equivalent circuit of Figure 6A at an angular frequency $\omega$, where $\omega=2\pi*$frequency, is given by:

$$Z = R_\infty + \frac{R_0 - R_\infty}{1 + (j\omega\tau)} \tag{2}$$

where:

$R_\infty$ = impedance at infinite applied frequency
$R_0$ = impedance at zero applied frequency = $R_e$ and,
$\tau$ is the time constant of the capacitive circuit.

However, the above represents an idealised situation which does not take into account the fact that the cell membrane is an imperfect capacitor. Taking this into account leads to a modified model in which:

$$Z = R_\infty + \frac{R_0 - R_\infty}{1 + (j\omega\tau)^{(1-\alpha)}} \tag{3}$$

where:

$\alpha$ has a value between 0 and 1 and can be thought of as an indicator of the deviation of a real system from the ideal model.

The values of impedance parameters $R_0$, $R_\infty$ or $Z_c$ may be determined in any one of a number of manners such as by:

- estimating values based on impedance measurements performed at selected respective frequencies;
- solving simultaneous equations based on the impedance values determined at different frequencies;
- using iterative mathematical techniques;
- extrapolation from a "Wessel plot" similar to that shown in Figure 6B;
- performing a function fitting technique, such as the use of a polynomial function.

For example, the Wessel plot is often used in BIS (Bioimpedance Spectroscopy) Bioimpedance Spectroscopy (BIS) devices, which perform multiple measurements over a range of frequencies, such as from 4 kHz to 1000 kHz, using 256 or more different frequencies within this range. A regression procedure is then used to fit the measured data to the theoretical semi-circular locus, allowing values for $R_\infty$ and $R_0$ to be calculated.

The regression analysis is computationally expensive, requiring a device with significant processing power to perform the calculations, which in turn results in relatively high power usage by the apparatus, requiring a larger battery, and adding to the weight and size of the apparatus.

A further issue is that a large number of data points are required to perform the regression analysis, and as measurements are typically performed at each frequency sequentially, the measurement process takes a significant amount of time, such as several seconds. This is undesirable as remaining still for long periods of time can cause discomfort for the subject. Additionally, the subject may move during the measurement procedure, which can affect the measured impedance values, for example due to changes in capacitive and/or inductive coupling between the subject and environment, leads and electrodes. This can lead to inaccuracies in the measured values.

A circle may be described by the equation:

$$(x - i)^2 + (y-j)^2 = r^2 \qquad\qquad (3)$$

where: i and j are the centre of the circle and r is the radius.

Additionally, a circle may be uniquely defined by the co-ordinates of three points $(x_{1-3}, y_{1-3})$ located on the locus, as shown in Figure 4. Accordingly, three simultaneous equations can be defined, one for each of three loci that describe the circle that fits these points, as shown by equations (4) below.

$$
\begin{aligned}
(x_1-i)^2 + (y_1 - j)^2 &= r^2 \\
(x_2-i)^2 + (y_2 - j)^2 &= r^2 \\
(x_3-i)^2 + (y_3 - j)^2 &= r^2
\end{aligned}
\qquad\qquad (4)
$$

Solving these three simultaneous equations allows calculation of the radius (r) and the co-ordinates of the centre of the circle (i, j). From these data, $R_0$ and $R_\infty$ are readily computed from geometric first principles.

Accordingly, this technique allows a value for $R_0$ and optionally $R_\infty$ to be derived in a computationally less expensive manner than if a regression analysis is performed. Additionally, this also requires a reduced number of data points. This allows a value of $R_0$ to be determined more rapidly, and with a more basic processor than can be achieved using BIS and regression analysis, which in turn renders the device required to determine a value of $R_0$ less expensive to manufacture.

In particular, this is achieved by performing impedance measurements at at least three frequencies. Indications of the signals are used to determine first and second impedance parameter values at each of the frequencies. The nature of the impedance parameter values will vary depending on the preferred implementation. Thus, for example the impedance parameter values could include magnitude and phase information relating to the measured signals. However, in one example the impedance parameter values are indicative of the resistance and reactance, as derived from the magnitude and phase signals.

Once this is completed, simultaneous equations are solved using the first and second impedance parameter values determined at each of the three frequencies, thereby allowing circle parameters to be determined. The circle parameters are used to define a locus corresponding to at least part of an arc of a circle in a space defined by the parameter values. Thus, in one example, the simultaneous equations represent a circular locus provided in a reactance/resistance space, similar to the Wessel plot described above.

Theoretical impedance parameter values, such as $R_0$ and $R_\infty$ can then be determined from the circle parameters.

One potential disadvantage of the use of simultaneous equations is that if one of the impedance measurements is inaccurate for any reason, this can lead to a large deviation in the calculated value of $R_0$. Accordingly, in one example, impedance measurements are performed at more than three frequencies, with circle parameters for all possible combinations of impedance measurements at three frequencies being calculated. The average can be provided along with the standard deviation as a measure of the goodness of fit of the data to the Cole model. In the event that one of the measurements is inaccurate, this can be accounted for by excluding one or more outlier measurements, such as measurements that deviates the greatest amount from the mean, or measurements differing by more than a set number of standard deviations from the mean, allowing the mean to be recalculated, thereby providing more accurate values.

Whilst this process uses additional measurements, such as four or five measurements, this is still significantly less than the 256 or more frequencies typically performed using a BIS measurement protocol, allowing the measurement process to be performed more quickly.

In one example, the frequencies used are in the range 0 kHz to 1000 kHz, and in one specific example, four measurements are recorded at frequencies of 25 kHz, 50 kHz, 100 kHz, and 200 kHz, although any suitable measurement frequencies can be used.

A further alternative for determining impedance parameter values such as $R_0$ and $R_\infty$ is it perform impedance measurements at a single frequency, and use these as an estimate of the parameter values. In this instance, measurements performed at a single low frequency can be used to estimate $R_0$, whilst measurements at a single high frequency can be used to estimate $R_\infty$.

The above described equivalent circuit models the resistivity as a constant value and does not therefore accurately reflect the impedance response of a subject, and in particular does not accurately model the change in orientation of the erythrocytes in the subject's blood stream, or other relaxation effects. To more successfully model the electrical conductivity of the human body, an improved CPE based model may alternatively be used.

In any event, it will be appreciated that any suitable technique for determination of the parameter values such as $R_0$, $Z_c$, and $R_\infty$ may be used.

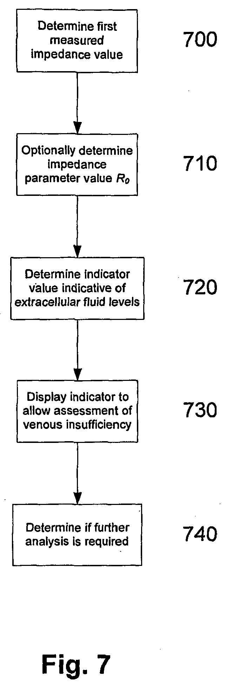

A first comparative example of a process for analysing impedance measurements to allow assessment of venous insufficiency will now be described with reference to Figure 7.

In this example, at step 700, at least one first impedance value is determined using the method described above. The measurement is typically performed with the subject in a specific orientation, such as in a supine or standing position. This is performed to either maximise or minimise the effect of blood pooling, and this will depend on the analysis performed.

In this first comparative example, the subject is made to stand for a set time period such as between five and fifteen minutes to maximize the effect of any blood pooling. In general, a marked increase in blood pooling is achieved after five minutes, with the blood levels reaching a relatively static maximum after approximately fifteen minutes. Accordingly, whilst it is preferable for the subject to stand for fifteen minutes to thereby maximise blood pooling, even after five minutes sufficient pooling occurs to allow measurements to be performed. It will be appreciated from this that the length of time selected may depend on factors such as the amount of time available for the measurement process and the ability of the subject to remain in standing position.

Furthermore, the subject may be required to lay in a supine position for a set time period, such as five to fifteen minutes prior to standing. This can be performed to minimise any blood pooling before standing, so as to provide a more accurate baseline status for the subject prior to measurements being performed. Again, a marked reduction in pooling is achieved after five minutes, with the level of pooling typically reaching a reasonably static minimum after approximately fifteen minutes, so the length of time used will depend on factors such as the amount of time available to make a measurement.

At step 710 an impedance parameter value $R_0$ is optionally determined. This can be performed if multiple impedance values are determined. Otherwise, a single impedance measurement can be made at a low frequency, such as below 10 kHz, as this provides a reasonably close approximation of $R_0$.

At step 720, an indicator that is indicative of the extracellular $R_e$ fluid levels within the subject is determined, with this being displayed to the user at step 730.

The indicator can be any form of suitable indicator such as a numerical value based on the value of the impedance parameter value $R_0$. The indicator may also be scaled to provide a numerical value that is indicative of the presence, absence or degree of venous insufficiency or oedema. The indicator can also be based on the results of a comparison of a numerical value to a reference, such as an oedema reference.

The oedema reference could be any suitable form of reference. Thus, in one example, the oedema reference can be based on an equivalent impedance parameter value determined for a different limb of the subject, such as an arm. This is possible, as, for a subject not suffering from venous insufficiency, there is a predictable relationship between the extracellular fluid levels between different limbs. Thus, for example, if the subject is suffering from a condition other than venous insufficiency, which causes a general change in extracellular fluid levels, then this should affect body segments in an assessable manner, thereby allowing venous insufficiency to be identified.

Minor variations in tissue may occur between different body segments in a healthy subject, and this can be accounted for by providing a tolerance to the comparison. Thus, for example, this could take into account naturally expected variations between different limbs in normal healthy subjects, for example due to limb dominance, previous analysis for the subject, or the like. The tolerance may also depend on a number of factors, such as the subject's age, weight, sex and height, and again a respective range can be selected based on these factors.

Alternatively, the oedema reference can be based on a reference derived from sample populations, or the like. The oedema reference can be selected based on the subject parameters, so that the value of the indicator is compared to values of the indicator derived from a study of a sample population of other individuals having similar subject parameters.

As a further alternative, the oedema reference can be based on a previously measured reference for the subject, for example determined before the subject suffered from venous insufficiency or oedema. This allows a longitudinal analysis to be performed, thereby allowing the onset or progression of venous insufficiency to be assessed.

The indicator can additionally and/or alternatively be displayed on a graphical linear or nonlinear scale, with the position of a pointer on the scale being indicative of extracellular fluid levels and or the presence, absence or degree of oedema or venous insufficiency. In one example, the linear scale can include thresholds at values representing ranges indicative of the presence or absence of oedema or venous insufficiency, as derived from sample population data, or other references.

At step 740, the user can use the indicator to assess whether further investigation is required. In this regard, a high extra-cellular fluid level indicative of the presence of oedema is a good indication that the subject has venous insufficiency, but this may need to be confirmed with further measurements, and/or analysis.

The above described example allows for a rapid assessment of the presence of venous insufficiency. This can be performed using BIA, which allows relatively simple apparatus and processing to be used, thereby reducing the cost of equipment required to assess venous insufficiency compared to more complex techniques. Despite this, the process is more reliable than current non-invasive techniques such as SPG and APG. In this regard, changes in fluid levels can typically be detected using impedance measurements before the fluid level changes have a noticeable impact on limb volume, thereby making the impedance measurement process more sensitive than other techniques such as SPG or APG.

Examples for performing further investigation will now be described in more detail.

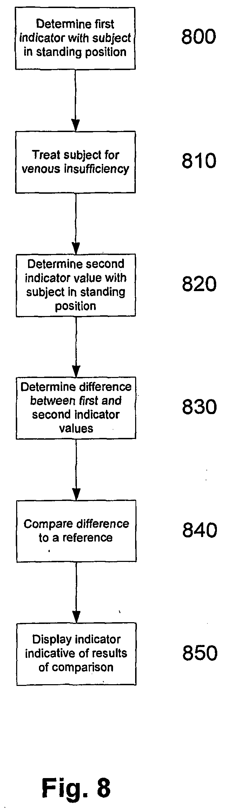

In the comparative example of Figure 8, at step 800 a first indicator value is determined with the subject in a standing position, as described with respect to Figure 7, to maximise blood pooling. This is performed before the subject is treated so that the first indicator acts as a pre-treatment indicator. At step 810, the subject is treated for venous insufficiency, by performing ablation, or the like. Following this, at step 820, a second indicator value is determined using a similar technique, (i.e. with the subject in a standing position) which acts as a post-treatment indicator.

The processing system 102 then determines any difference between the first and second indicator values at step 830, with the difference being compared to a reference, such as a treatment reference at step 840, thereby allowing the relevance of any change to be assessed. If the comparison indicates that there is a reduction in the extra-cellular fluid levels greater than a threshold amount, then this indicates that the treatment is successful or has at least had an impact, thereby allowing the presence of venous insufficiency to be confirmed at step 850. The magnitude of any difference may also be used to determine the degree of any venous insufficiency, and/or the effectiveness of the treatment.

Again, the treatment reference can be derived in any one of a number of manners. For example, treatment reference can be obtained from data collected from a sample population of subjects, which acts as a pool of data from which normalised expected differences for successfully treated, untreated and/or healthy subjects can be determined. The treatment reference is then generated by selecting reference values that are determined to be relevant to the test subject based on the subject parameters such as age, sex, height, weight, race, interventions, or the like.

In this instance, given a preliminary indication that venous insufficiency is present following the procedure of Figure 7, treatment of the subject is performed, with the presence of venous insufficiency being confirmed if the treatment is successful in the sense that it results in a reduction in extra-cellular fluid levels. This therefore allows a more reliable assessment of venous insufficiency, but advantageously also simultaneously treats the venous insufficiency, ensuring that the subject is treated as rapidly as possible, without having to await further analysis. In the instance that the subject does not have venous insufficiency, then there is no negative effect of performing the treatment. It will also be appreciated that this allows the analysis to be confirmed using the same apparatus as used to perform the initial assessment, thereby simplifying the analysis for the relevant health professional performing the assessment.

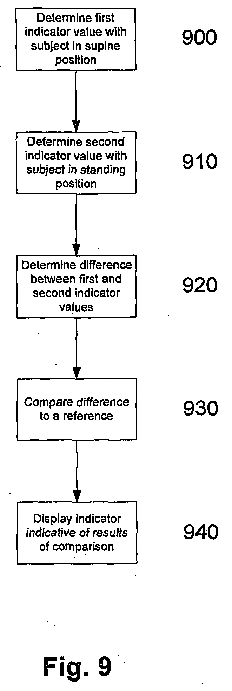

In the specific example of Figure 9, at step 900 a first indicator value is determined with the subject in a supine position. This measurement is typically performed after the subject has been allowed to rest for some set time, such as five to fifteen minutes. This reduces the effect of any blood pooling, allowing a baseline reading to be established. The subject then stands for a predetermined time period, such as five to fifteen minutes, to maximize blood pooling before a second indicator value is determined using the technique described above, at step 910. Again, the first and second indicator values are indicative of extra-cellular fluid levels, and therefore could be based on one or more low frequency impedance measurements or the impedance parameter value $R_0$, as derived from impedance measurements in some manner. At step 920, the processing system 102 then determines any difference between the first and second indicator values, with the difference being compared to a reference, such as a pooling reference, at step 930.

Again, the pooling reference can be based on similar indicator values derived from sample populations, or the like. Alternatively, the pooling reference can be based on first and second indicator values previously determined for the subject, for example prior to the onset of venous insufficiency, allowing longitudinal analysis to be performed.

In one example, the reference is determined as a percentage change from the baseline reading, so that a change of less than a predetermined amount, such as 15%, indicates that the subject does not have venous insufficiency. For a percentage change greater than the predetermined amount, such as 15%, this indicates that the level of pooling is abnormal, and hence that the subject may have venous insufficiency, with the magnitude of the percentage change being indicative of the degree of venous insufficiency. Thus, for example, a change of greater than 20 %, would typically be indicative of venous insufficiency.

Results of the comparison can be displayed at step 940, to allow the relevance of any change to be assessed. In this regard, if the comparison indicates that there is an increase in the extra-cellular fluid levels greater than an amount determined from the pooling reference, then this indicates that there is significant blood pooling within the subject, which is in turn indicative of venous insufficiency. It will be appreciated from this, that the magnitude of the difference between the first and second indicator values can be indicative of the degree of venous insufficiency.

In this instance, given a preliminary indication that venous insufficiency is present following the procedure of Figure 7, the subject is reorientated and the measurement re-performed. In this regard, if the indicator derived in the process of Figure 7 is made in the standing position, then this can act as the second indicator, with the subject being reorientated to allow the first indicator to be determined. Accordingly, it will be appreciated that the terms "first" and "second" are used to identify respective indicators and do not necessarily imply or require an order to the measurements.

However, in general the first indicator is measured first to allow an accurate baseline to be established, although this is not essential. This allows a more reliable assessment of venous insufficiency than achievable with prior art techniques, and allows the analysis to be confirmed using the same apparatus as used to perform the initial assessment. This also allows assessment to be confirmed without requiring treatment of the subject, which can reduce the cost burden of the assessment.

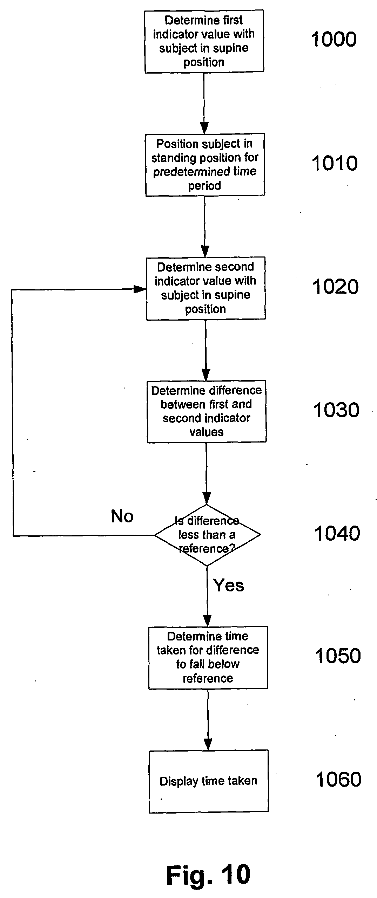

In the specific example of Figure 10, at step 1000 a first indicator value is determined with the subject in a supine position. As in the previous example, this is performed to allow a baseline reading to be established. The subject then stands for a predetermined time period, such as five to fifteen minutes, to maximize blood pooling at step 1010, before returning to a supine position to allow a second indicator value to be determined using the technique described above, at step 1020.

At step 1030, the processing system 102 then determines any difference between the first and second indicator values, before determining if the difference is below a reference, such as a return reference, at step 1040. If not, the process returns to step 1020, allowing a new second indicator to be determined.

The reference may be derived from sample populations, previous measurements for the subject, or the like. Alternatively, the reference is deemed to be a certain percentage variation from the baseline reading. Thus, in this example, the time taken to return to within 5-10% of the baseline reading can be determined.

Steps 1020 to 1040 are repeated until the difference between the first and second indicators is below the return reference. At step 1050, the length of time taken for the difference to fall below the pooling reference is determined, with this being displayed to the user at step 1060.

If the time taken to return to the baseline (or the reference), is less than a reference amount, such as fifteen minutes, then this is indicative that the subject does not have venous insufficiency. However, if the time taken is greater than the reference time, then this is indicative that the subject does have venous insufficiency.

In this regard, in a healthy subject, the amount of blood will return to the baseline amount relatively quickly, whilst in a subject with venous insufficiency, this can take several minutes. Accordingly, the time taken for blood pooling in the leg to be reduced is in turn indicative of the presence, absence or degree of venous insufficiency. The return reference is used to take into account natural variation and to allow the measurement to be performed in a reasonable time period.

In this instance, by measuring the time taken for the subject to return to a baseline reading, this provides a direct correlation for the ability of the venous system to counteract any blood pooling, which is directly indicative of the degree of venous insufficiency. This therefore provides an accurate technique for assessing the severity of venous insufficiency without requiring treatment or complex apparatus.

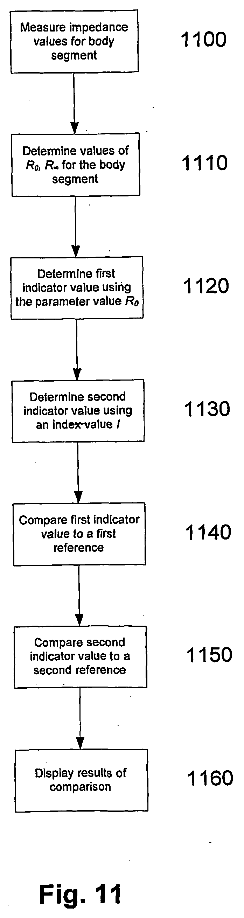

An example of a process for allowing oedema and lymphoedema to be distinguished will now be described with reference to Figure 11. In one example, this process can be used to help assess the presence of venous insufficiency. For example, if measurements are performed on the subject's leg, and it is determined that the subject has oedema but not lymphoedema, then this is indicative that the subject has venous insufficiency. Alternatively however, this technique can be used to distinguish between oedema and lymphoedema in other body segments, such as arms. This can be used in helping to identify optimum management programmes depending on the conditions suffered by the subject.

Accordingly, for the purpose of this example, the technique will be described as being generally applied to a body segment to allow oedema and lymphoedema to be distinguished. It will be appreciated that in the event that this technique is used for identifying venous insufficiency, then the process will be performed on the subject's leg, but that the process could also be applied to other body segments.

In the specific example of Figure 11, at step 1100 impedance values are determined for the body segment at a number of different frequencies. If this technique is being used to determine indicators that can be used in identifying venous insufficiency, then these measurements are typically performed with the subject in a standing position to maximise blood pooling.

At step 1110, the processing system 102 determines values for the impedance parameters $R_0$, $R_\infty$ as described in more detail above. Thus, this could be performed using regression analysis applied to BIS measurements. Alternatively, the process could use MFBIA, and solving of simultaneous equations to determine the impedance parameter values. Other techniques could also be used as appropriate in which combinations of individual measurements at selected frequencies are used. Thus, for example, single measurements at low and high frequencies respectively could be used as an estimate of $R_0$ and $R_\infty$.

At step 1120, a first indicator value is determined using the impedance parameter value $R_0$. It will be appreciated that this can alternatively be derived from a single low frequency impedance measurement described above, although in another example, measurements at multiple frequencies can be used for subsequent steps, in which case the need to make additional single frequency measurements can be avoided.

At step 1130, an index is determined for the body segment which provides an indication of the distribution between intra and extracellular fluids. In one example, the index is indicative of the ratio of extracellular fluid to intracellular fluid $R_i/R_e$. In this regard, using the values for the extracellular fluid resistance $R_e$ and intracellular fluid resistance $R_i$ above, the index $I$ is given by the equation:

$$I = \frac{R_i}{R_e} = \frac{R_\infty}{R_0 - R_\infty} \tag{4}$$

At step 1140, the first indicator value is compared to a first reference, such as the oedema reference described above, with the results of the comparison allowing the presence of oedema to be determined.

At step 1150, the index value is compared to a second reference, with the results of the comparison allowing determination of whether the subject has lymphoedema. The second reference can be determined in any one of a number of manners.

In one example, the index is compared to a reference index value determined for another limb. This is possible, as, for a subject not having lymphoedema, there is generally a degree of similarity of intra- and extra-cellular fluid levels, even between different body segments. Typically minor variations in tissue will occur between different body segments, for example due to inherent differences between different types of limb, or due to limb dominance, and this can be accounted for using appropriate tolerances in a manner similar to that described above. Additionally, and/or alternatively, different references can be used, such as references derived from sample populations, previous measurements made for the subject, or the like.

The results of the comparison can be displayed to the user to allow assessment of whether the subject has oedema and/or lymphoedema.

In the event that measurements are performed on the subject's leg, whilst the subject is in a standing position, and this indicates that the subject is likely to have oedema which is not lymphoedema, then this is indicative that the subject has venous insufficiency. In this instance, the magnitude of the indicator and index values can be used to assess the degree of venous insufficiency. Alternatively, this technique can be used on other parts of the body to distinguish between oedema and lymphoedema.

In a further example, the above described process can be repeated with the subject in supine and standing positions. In this instance, changes in the indicator and index values can further assist in assessing the presence, absence or degree of venous insufficiency.

In this example, whilst more complex BIS apparatus is required, this can provide a more accurate indication of venous insufficiency severity than can be achieved using the prior art or other techniques. Furthermore, by using the BIS apparatus described above, the assessment can be performed rapidly, without requiring the subject to undergo exercise regimes, which may not be appropriate for some subjects.

In the above described examples, the term impedance generally refers to a measured impedance value or impedance parameter value derived therefrom. The term resistance refers to any measured value relating to the impedance, such as admittance of reactance measurements.

The term processing system is intended to include any component capable of performing processing and can include any one or more of a processing system and a computer system.

Persons skilled in the art will appreciate that numerous variations and modifications will become apparent. All such variations and modifications which become apparent to persons skilled in the art, should be considered to fall within the scope of the invention as defined in the claims.

Features from different examples above may be used interchangeably where appropriate. Thus, for example, multiple different indicators may be determined and compared to respective thresholds.

Furthermore, whilst the above examples have focussed on a subject such as a human, it will be appreciated that the measuring device and techniques described above can be used with any animal, including but not limited to, primates, livestock, performance animals, such race horses, or the like.

The above described processes can be used in determining biological indicators, which in turn can be used for diagnosing the presence, absence or degree of a range of conditions and illnesses, including, but not limited to oedema, lymphodema, body composition, or the like.

Furthermore, whilst the above described examples have focussed on the application of a voltage signal to cause a current to flow through the subject, this is not essential and the process can also be used when applying a current signal.

It will also be appreciated that the term impedance measurement covers admittance and other related measurements.

**Claims**

1. Apparatus for use in analysing impedance measurements performed on a subject, the apparatus including a processing system (102) operable to:

    a) determine first and second impedance values indicative of the impedance of at least one leg segment of the subject;
    b) determine an indicator using the first and second impedance values, the indicator being indicative of extracellular fluid levels in the at least one leg segment and for use in the assessment of venous insufficiency; the processing system being operable to determine the indicator by:

        i) determining a first indicator value with the subject in a first orientation using the first impedance value;
        ii) after positioning the subject in a second orientation for a predetermined time period, determining a second indicator value with the subject in the first orientation using the second impedance value; and,
        iii) determining the indicator using a difference between the first and second indicator values, **characterised in that** the processing system is operable to:

            (a) monitor the difference;
            (b) determine the time taken for the difference to fall below a reference; and,
            (c) provide an indication of the time taken to allow determination of a presence, absence or degree of venous insufficiency.

2. Apparatus according to claim 1, wherein the processing system (102) is operable to:

    a) compare the indicator to a reference; and,
    b) provide an indication of the results of the comparison to allow determination of a presence, absence or degree of venous insufficiency.

3. Apparatus according to claim 1, wherein the processing system (102) is operable to determine an index using at least one impedance value, the index being indicative of a ratio of extracellular to intracellular fluid levels in the at least leg segment, the index being usable in the assessment of venous insufficiency.

4. Apparatus according to claim 3, wherein the processing system (102) is operable to:

    a) compare the index to a reference; and,
    b) provide an indication of the results of the comparison to allow determination of a presence, absence or degree of venous insufficiency.

5. Apparatus according to claim 3, wherein the processing system (102) is operable to:

    a) determine a first index value with the subject in a first orientation;
    b) determine a second index value with the subject in a second orientation; and,
    c) determine an index change based on a difference between the first and second fluid index values, the index change being usable in the assessment of venous insufficiency.

6. Apparatus according to claim 1, wherein the processing system (102) is operable to:

a) determine a pre-treatment indicator value prior to treatment of the subject;
b) determine a post-treatment indicator value following treatment of the subject for venous insufficiency; and,
c) determine an indicator change based on a difference between the pre-treatment and post-treatment indicator values, the indicator change being usable in the assessment of venous insufficiency.

7. Apparatus according to any one of the claims 1 to 6, wherein the processing system is operable to use a reference that is at least one of:

a) an indicator or index determined for another limb of the subject;
b) a reference determined from a sample population; and,
c) a previous indicator or index determined for the subject.

8. Apparatus according to any one of the claims 1 to 7, wherein the processing system (102) is operable to display at least one of:

a) an indicator;
b) an index ratio;
c) an index;
d) an indicator change;
e) an index change;
f) one or more impedance parameter values; and,
g) results of a comparison.

9. Apparatus according to any one of the claims 1 to 8, wherein the processing system (102) is operable to use the first and second impedance measurements as an estimate of a resistance of the subject at a zero measurement frequency and wherein the first and second impedance values are measured at a measurement frequency of at least one of:

a) less than 100 kHz;
b) less than 50 kHz; and,
c) less than 10 kHz.

10. Apparatus according to any one of the claims 1 to 9, wherein the processing system (102) is operable to use the first and second impedance measurements as an estimate of a resistance of the subject at an infinite measurement frequency and wherein the first and second impedance values are measured at a measurement frequency of at least one of:

a) greater than 200 kHz;
b) greater than 500 kHz; and,
c) greater than 1000 kHz.

11. Apparatus according to any one of the claims 1 to 10, wherein the processing system (102) is operable to:

a) determine a plurality of first and second impedance values; and,
b) determine at least one impedance parameter value from the plurality of first and second impedance values.

12. Apparatus according to claim 11, wherein the impedance parameter values include at least one of:

$R_0$ which is the resistance at zero frequency;
$R_\infty$ which is the resistance at infinite frequency; and,
$Z_c$ which is the resistance at a characteristic frequency.

13. Apparatus according to any one of the claims 1 to 12, wherein the processing system is operable to cause the impedance measurements to be performed.

14. Apparatus according to claim 13, wherein the processing system is operable to:

a) cause one or more electrical signals to be applied to the subject using a first set of electrodes (113A, 113B);
b) measure electrical signals across a second set of electrodes (115A, 115B) applied to the subject in response to the applied one or more signals; and,
c) determine from the applied signals and the measured signals at least one measured impedance value.

15. Apparatus according to claim 14, wherein the apparatus includes:

a) a signal generator (117A, 117B) operable to generate electrical signals; and,
b) a sensor (118A, 118B) operable to sense electrical signals.

16. Apparatus according to claim 1, wherein the processing system (102) operable to:

a) determine at least one impedance value indicative of the impedance of at least one body segment of the subject;
b) determine an indicator using at least one impedance value, the indicator being indicative of extracellular fluid levels in the at least one body segment;
c) determine an index using at least one impedance value, the index being indicative of a ratio of extracellular to intracellular fluid levels in the at least one body segment;
d) compare the indicator to a first reference;
e) compare the index to a second reference; and,
f) provide an indication of the results of the comparisons.

17. A method for use in analysing impedance measurements performed on a subject, the method including, in a processing system (102):

a) determining first and second impedance values indicative of the impedance of at least one leg segment of the subject;
b) determining an indicator using the first and second impedance values, the indicator being indicative of extracellular fluid levels in the at least one leg segment and being usable in the assessment of venous insufficiency; and wherein the method further includes, in the processing system determining the indicator by:

i) determining a first indicator value with the subject in a first orientation using the first impedance value;
ii) after positioning the subject in a second orientation for a predetermined time period, determining a second indicator value with the subject in the first orientation using the second impedance value; and,
iii) determining the indicator using the difference between the first and second fluid indicator values, **characterised in that** the method further comprises, in the processing system:

(a) monitor the difference;
(b) determine the time taken for the difference to fall below a reference; and,
(c) provide an indication of the time taken to allow determination of a presence, absence or degree of venous insufficiency.

**Patentansprüche**

1. Gerät für den Gebrauch bei der Analyse von Impedanzmessungen, die an einem Subjekt ausgeführt werden, wobei das Gerät ein Verarbeitungssystem (102) aufweist, das betreibbar ist, um:

a) einen ersten und einen zweiten Impedanzwert zu bestimmen, die die Impedanz mindestens eines Beinsegments des Subjekts angeben,
b) einen Indikator zu bestimmen, indem der erste und der zweite Impedanzwert verwendet werden, wobei der Indikator extrazellulare Fluidniveaus in dem mindestens einen Beinsegment angibt, und für den Gebrauch bei der Beurteilung venöser Insuffizienz, wobei das Verarbeitungssystem betreibbar ist, um den Indikator zu bestimmen durch:

i) Bestimmen eines ersten Indikatorwerts mit dem Subjekt in einer ersten Ausrichtung, indem der erste Impedanzwert verwendet wird,
ii) nach dem Positionieren des Subjekts in einer zweiten Ausrichtung während einer vorbestimmten Zeitspanne, Bestimmen eines zweiten Indikatorwerts mit dem Subjekt in der ersten Ausrichtung, indem der zweite Impedanzwert verwendet wird, und

iii) Bestimmen des Indikators, indem ein Unterschied zwischen dem ersten und dem zweiten Indikatorwert verwendet wird, **dadurch gekennzeichnet, dass** das Verarbeitungssystem betreibbar ist, um:

(a) den Unterschied zu überwachen,
(b) die Zeit zu bestimmen, die erforderlich ist, damit der Unterschied unter eine Referenz fällt, und
(c) eine Angabe der Zeit bereitzustellen, die erforderlich ist, um das Bestimmen einer Gegenwart, Abwesenheit oder eines Grads venöser Insuffizienz zu erlauben.

2. Gerät nach Anspruch 1, wobei das Verarbeitungssystem (102) betreibbar ist, um:

a) den Indikator mit einer Referenz zu vergleichen, und
b) eine Angabe der Resultate des Vergleichs bereitzustellen, um das Bestimmen einer Gegenwart, Abwesenheit oder eines Grads venöser Insuffizienz zu erlauben.

3. Gerät nach Anspruch 1, wobei das Verarbeitungssystem (102) betreibbar ist, um einen Index zu bestimmen, indem mindestens ein Impedanzwert verwendet wird, wobei der Index ein Verhältnis extrazellularer zu intrazellularer Fluidniveaus in dem mindestens einen Beinsegment angibt, wobei der Index bei der Beurteilung venöser Insuffizienz verwendbar ist.

4. Gerät nach Anspruch 3, wobei das Verarbeitungssystem (102) betreibbar ist, um:

a) den Index mit einer Referenz zu vergleichen, und
b) eine Angabe der Resultate des Vergleichs bereitzustellen, um das Bestimmen einer Gegenwart, Abwesenheit oder eines Grads venöser Insuffizienz zu erlauben.

5. Gerät nach Anspruch 3, wobei das Verarbeitungssystem (102) betreibbar ist, um:

a) einen ersten Indexwert mit dem Subjekt in einer ersten Ausrichtung zu bestimmen,
b) einen zweiten Indexwert mit dem Subjekt in einer zweiten Ausrichtung zu bestimmen, und
c) einen Indexwechsel basierend auf einem Unterschied zwischen dem ersten und dem zweiten Fluidindexwert zu bestimmen, wobei der Indexwechsel bei der Beurteilung venöser Insuffizienz verwendbar ist.

6. Gerät nach Anspruch 1, wobei das Verarbeitungssystem (102) betreibbar ist, um

a) einen Vorbehandlungsindikatorwert vor der Behandlung des Subjekts zu bestimmen,
b) einen Nachbehandlungsindikatorwert im Anschluss an die Behandlung des Subjekts für venöse Insuffizienz zu bestimmen, und
c) einen Indikatorwechsel basierend auf einem Unterschied zwischen dem Vorbehandlungsindikatorwert und dem Nachbehandlungsindikatorwert zu bestimmen, wobei der Indikatorwechsel bei der Beurteilung venöser Insuffizienz verwendbar ist.

7. Gerät nach einem der Ansprüche 1 bis 6, wobei das Verarbeitungssystem betreibbar ist, um eine Referenz zu verwenden, die:

a) ein Indikator oder Index ist, der für eine andere Gliedmaße des Subjekts bestimmt wird, und/oder
b) eine Referenz ist, die aus einer Bevölkerungsstichprobe bestimmt wird, und/oder
c) ein vorhergehender Indikator oder Index ist, der für das Subjekt bestimmt wurde.

8. Gerät nach einem der Ansprüche 1 bis 7, wobei das Verarbeitungssystem (102) betreibbar ist, um Folgendes anzuzeigen:

a) einen Indikator und/oder
b) ein Indexverhältnis und/oder
c) einen Index und/oder
d) einen Indikatorwechsel und/oder
e) einen Indexwechsel und/oder

f) einen oder mehrere Impedanzparameterwerte und/oder

g) Resultate eines Vergleichs.

9. Gerät nach einem der Ansprüche 1 bis 8, wobei das Verarbeitungssystem (102) betreibbar ist, um die erste und die zweite Impedanzmessung als eine Schätzung eines Widerstands des Subjekts bei einer Messfrequenz gleich null zu verwenden, und wobei der erste und der zweite Impedanzwert bei einer Messfrequenz von:

a) weniger als 100 kHz und/oder

b) weniger als 50 kHz und/oder

c) weniger als 10 kHz gemessen werden.

10. Gerät nach einem der Ansprüche 1 bis 9, wobei das Verarbeitungssystem (102) betreibbar ist, um die erste und die zweite Impedanzmessung als eine Schätzung eines Widerstands des Subjekts bei einer unendlichen Messfrequenz zu verwenden, und wobei der erste und der zweite Impedanzwert bei einer Messfrequenz von:

a) größer als 200 kHz und/oder

b) größer als 500 kHz und/oder

c) größer als 1000 kHz gemessen werden.

11. Gerät nach einem der Ansprüche 1 bis 10, wobei das Verarbeitungssystem (102) betreibbar ist, um:

a) mehrere erste und zweite Impedanzwerte zu bestimmen, und

b) mindestens einen Impedanzparameterwert aus den mehreren ersten und zweiten Impedanzwerten zu bestimmen.

12. Gerät nach Anspruch 11, wobei die Impedanzparameterwerte aufweisen:

$R_0$, der der Widerstand bei null Frequenz ist und/oder

$R_\infty$, der der Widerstand bei unendlicher Frequenz ist und/oder

$Z_C$, der der Widerstand bei einer charakteristischen Frequenz ist.

13. Gerät nach einem der Ansprüche 1 bis 12, wobei das Verarbeitungssystem betreibbar ist, um das Ausführen der Impedanzmessungen zu veranlassen.

14. Gerät nach Anspruch 13, wobei das Verarbeitungssystem betreibbar ist, um:

a) das Anlegen eines oder mehrerer elektrischer Signale an das Subjekt unter Verwenden eines ersten Satzes von Elektroden (113A, 113B) zu veranlassen,

b) elektrische Signale über einen zweiten Satz von Elektroden (115A, 115B), die an das Subjekt angelegt werden, als Reaktion auf das eine oder die mehreren angelegten Signale zu messen, und

c) aus den angelegten Signalen und den gemessenen Signalen mindestens einen gemessenen Impedanzwert zu bestimmen.

15. Gerät nach Anspruch 14, wobei das Gerät Folgendes aufweist:

a) einen Signalgenerator (117A, 117B), der betreibbar ist, um elektrische Signale zu erzeugen, und

b) einen Sensor (118A, 118B), der betreibbar ist, um elektrische Signale zu erfassen.

16. Gerät nach Anspruch 1, wobei das Verarbeitungssystem (102) betreibbar ist, um

a) mindestens einen Impedanzwert zu bestimmen, der die Impedanz mindestens eines Körpersegments des Subjekts angibt,

b) einen Indikator zu bestimmen, indem mindestens ein Impedanzwert verwendet wird, wobei der Indikator extrazellulare Fluidniveaus in dem mindestens einen Körpersegment angibt,

c) einen Index zu bestimmen, indem mindestens ein Impedanzwert verwendet wird, wobei der Index ein Verhältnis extrazellularer zu intrazellularen Fluidniveaus in dem mindestens einen Körpersegment angibt,

d) den Indikator mit einer ersten Referenz zu vergleichen,

e) den Index mit einer zweiten Referenz zu vergleichen, und

f) eine Angabe der Resultate der Vergleiche bereitzustellen.

17. Verfahren für den Gebrauch bei der Analyse von Impedanzmessungen, die an einem Subjekt ausgeführt werden, wobei das Verfahren in einem Verarbeitungssystem (102) Folgendes aufweist:

a) Bestimmen eines ersten und eines zweiten Impedanzwerts, die die Impedanz mindestens eines Beinsegments des Subjekts angeben,
b) Bestimmen eines Indikators unter Verwenden des ersten und des zweiten Impedanzwerts, wobei der Indikator extrazellulare Fluidniveaus in dem mindestens einen Beinsegment angibt und bei der Beurteilung venöser Insuffizienz verwendbar ist, und wobei das Verfahren ferner in dem Verarbeitungssystem das Bestimmen des Indikators durch Folgendes aufweist:

i) Bestimmen eines ersten Indikatorwerts mit dem Subjekt in einer ersten Ausrichtung unter Verwenden des ersten Impedanzwerts,
ii) nach dem Positionieren des Subjekts in einer zweiten Ausrichtung während einer vorbestimmten Zeitspanne, Bestimmen eines zweiten Indikatorwerts mit dem Subjekt in der ersten Ausrichtung unter Verwenden des zweiten Impedanzwerts, und
iii) Bestimmen des Indikators unter Verwenden des Unterschieds zwischen dem ersten und dem zweiten Fluidindikatorwert, **dadurch gekennzeichnet, dass** das Verfahren ferner in dem Verarbeitungssystem Folgendes umfasst:

(a) Überwachen des Unterschieds,
(b) Bestimmen der Zeit, die erforderlich ist, damit der Unterschied unter eine Referenz fällt, und
(c) Bereitstellen einer Angabe der Zeit, die erforderlich ist, um das Bestimmen einer Gegenwart, Abwesenheit oder eines Grads venöser Insuffizienz zu erlauben.


**Revendications**

1. Appareil destiné à être utilisé dans l'analyse de mesures d'impédance effectuées sur un sujet, l'appareil comprenant un système de traitement (102) utilisable pour :

a) déterminer des première et seconde valeurs d'impédance indicatives de l'impédance d'au moins un segment de jambe du sujet ;
b) déterminer un indicateur en utilisant les première et seconde valeurs d'impédance, l'indicateur étant indicatif de niveaux de fluides extracellulaires dans l'au moins un segment de jambe et étant utilisé dans l'évaluation de l'insuffisance veineuse ; le système de traitement étant utilisable pour déterminer l'indicateur en :

i) déterminant une première valeur d'indicateur avec le sujet dans une première orientation en utilisant la première valeur d'impédance ;
ii) après avoir positionné le sujet dans une seconde orientation pendant une période de temps prédéterminée, en déterminant une seconde valeur d'indicateur avec le sujet dans la première orientation en utilisant la seconde valeur d'impédance ; et
iii) en déterminant l'indicateur en utilisant une différence entre les première et seconde valeurs d'indicateur, **caractérisé en ce que** le système de traitement est utilisable pour :

(a) surveiller la différence ;
(b) déterminer le temps pris par la différence pour chuter au-dessous d'une référence ; et
(c) fournir une indication du temps pris pour permettre la détermination d'une présence, d'une absence ou d'un degré d'insuffisance veineuse.

2. Appareil selon la revendication 1, dans lequel le système de traitement (102) est utilisable pour :

a) comparer l'indicateur à une référence ; et,
b) fournir une indication des résultats de la comparaison pour permettre la détermination d'une présence, d'une absence ou d'un degré d'insuffisance veineuse.

3. Appareil selon la revendication 1, dans lequel le système de traitement (102) est utilisable pour déterminer un index en utilisant au moins une valeur d'impédance, l'index étant indicatif d'un rapport de niveaux de fluides extracellulaires sur des niveaux de fluides intracellulaires dans l'au moins un segment de jambe, l'index étant utilisable dans l'évaluation d'une insuffisance veineuse.

4. Appareil selon la revendication 3, dans lequel le système de traitement (102) est utilisable pour :

a) comparer l'index à une référence ; et,
b) fournir une indication des résultats de la comparaison pour permettre la détermination d'une présence, d'une absence ou d'un degré d'insuffisance veineuse.

5. Appareil selon la revendication 3, dans lequel le système de traitement (102) est utilisable pour :

i) déterminer une première valeur d'index avec le sujet dans une première orientation ;
ii) déterminer une seconde valeur d'index avec le sujet dans une seconde orientation ; et
iii) déterminer un changement d'index basé sur une différence entre les première et seconde valeurs d'index de fluide, le changement d'index étant utilisable dans l'évaluation d'une insuffisance veineuse.

6. Appareil selon la revendication 1, dans lequel le système de traitement (102) est utilisable pour :

i) déterminer une valeur d'indicateur de prétraitement avant le traitement du sujet ;
ii) déterminer une valeur d'indicateur de post-traitement après le traitement du sujet pour insuffisance veineuse ; et
iii) déterminer un changement d'indicateur basé sur une différence entre les valeurs d'indicateur prétraitement et post-traitement, le changement d'indicateur étant utilisable dans l'évaluation d'une insuffisance veineuse.

7. Appareil selon l'une quelconque des revendications 1 à 6, dans lequel le système de traitement est utilisable pour utiliser une référence qui est au moins une valeur parmi les suivantes :

a) un indicateur ou un index déterminé pour un autre membre du sujet ;
b) une référence déterminée à partir d'un échantillon de population ; et
c) un précédent indicateur ou index déterminé pour le sujet.

8. Appareil selon l'une quelconque des revendications 1 à 7, dans lequel le système de traitement (102) est utilisable pour afficher au moins une valeur parmi les suivantes :

a) un indicateur ;
b) un rapport d'index ;
c) un index ;
d) un changement d'indicateur ;
e) un changement d'index ;
f) une ou plusieurs valeurs de paramètre d'impédance ; et
g) des résultats d'une comparaison.

9. Appareil selon l'une quelconque des revendications 1 à 8, dans lequel le système de traitement (102) est utilisable pour utiliser les première et seconde mesures d'impédance comme une estimation d'une résistance du sujet à une fréquence de mesure nulle et où les première et seconde valeurs d'impédance sont mesurées à une fréquence de mesure satisfaisant au moins une des conditions suivantes :

a) inférieure à 100 kHz ;
b) inférieure à 50 kHz ; et
c) inférieure à 10 kHz.

10. Appareil selon l'une quelconque des revendications 1 à 9, dans lequel le système de traitement (102) est utilisable pour utiliser les première et seconde mesures d'impédance comme une estimation d'une résistance du sujet à une fréquence de mesure infinie et où les première et seconde valeurs d'impédance sont mesurées à une fréquence de mesure satisfaisant au moins une des conditions suivantes :

a) supérieure à 200 kHz ;

b) supérieure à 500 kHz ; et

c) supérieure à 1000 kHz.

11. Appareil selon l'une quelconque des revendications 1 à 10, dans lequel le système de traitement (102) est utilisable pour :

a) déterminer une pluralité de première et seconde valeurs d'impédance ; et,

b) déterminer au moins une valeur de paramètre d'impédance à partir de la pluralité de première et seconde valeurs d'impédance.

12. Appareil selon la revendication 11, dans lequel les valeurs de paramètre d'impédance comprennent au moins une valeur parmi les suivantes :

$R_0$ qui est la résistance à une fréquence nulle ;

$R_\infty$ qui est la résistance à une fréquence infinie ; et,

$Z_c$ qui est la résistance à une fréquence caractéristique.

13. Appareil selon l'une quelconque des revendications 1 à 12, dans lequel le système de traitement est utilisable pour amener les mesures d'impédance à être effectuées.

14. Appareil selon la revendication 13, dans lequel le système de traitement est utilisable pour :

a) amener un ou plusieurs signaux électriques à être appliqués au sujet au moyen d'un premier jeu d'électrodes (113A, 113B) ;

b) mesurer les signaux électriques à travers un second jeu d'électrodes (115A, 115B) appliqués au sujet en réponse à l'un ou aux signaux appliqués ;

c) déterminer, à partir des signaux appliqués et des signaux mesurés, au moins une valeur d'impédance mesurée.

15. Appareil selon la revendication 14, dans lequel l'appareil comprend :

a) un générateur de signaux (117A, 117B) utilisable pour générer des signaux électriques ; et,

b) un capteur (118A, 118B) utilisable pour détecter des signaux électriques.

16. Appareil selon la revendication 1, dans lequel le système de traitement (102) est utilisable pour :

a) déterminer au moins une valeur d'impédance indicative de l'impédance d'au moins un segment corporel du sujet ;

b) déterminer un indicateur en utilisant au moins une valeur d'impédance, l'indicateur étant indicatif de niveaux de fluides extracellulaires dans l'au moins un segment corporel ;

c) déterminer un index en utilisant au moins une valeur d'impédance, l'index étant indicatif d'un rapport de niveaux de fluides extracellulaires sur des niveaux de fluides intracellulaires dans l'au moins un segment corporel ;

d) comparer l'indicateur à une première référence ;

e) comparer l'index à une seconde référence ; et,

f) fournir une indication des résultats des comparaisons.

17. Procédé destiné à être utilisé dans l'analyse de mesures d'impédance effectuées sur un sujet, le procédé comprenant, un système de traitement (102), les étapes suivantes :

a) déterminer des première et seconde valeurs d'impédance indicatives de l'impédance d'au moins un segment de jambe du sujet ;

b) déterminer un indicateur en utilisant les première et seconde valeurs d'impédance, l'indicateur étant indicatif de niveaux de fluides extracellulaires dans l'au moins un segment de jambe et étant utilisable dans l'évaluation de l'insuffisance veineuse ; et où le procédé comprend en outre, dans le système de traitement, de déterminer l'indicateur en :

i) déterminant une première valeur d'indicateur avec le sujet dans une première orientation en utilisant la première valeur d'impédance ;

ii) après avoir positionné le sujet dans une seconde orientation pendant une période de temps prédéterminée, en déterminant une seconde valeur d'indicateur avec le sujet dans la première orientation en utilisant la seconde valeur d'impédance ; et

iii) en déterminant l'indicateur en utilisant une différence entre les premières et seconde valeurs d'indicateur, **caractérisé en ce que** le procédé comprend en outre, dans le système de traitement, les étapes suivantes :

(a) surveiller la différence ;

(b) déterminer le temps pris par la différence pour chuter au-dessous d'une référence ; et

(c) fournir une indication du temps pris pour permettre la détermination d'une présence, d'une absence ou d'un degré d'insuffisance veineuse.

**Fig. 1**

Fig. 2

**Fig. 3**

**Fig. 4**

| | |
|---|---|
| Place electrodes on the subject | 500 |
| Select impedance measurement type using computer system 310 | 510 |
| The processing system 330 selects next measurement frequency $f_i$ | 520 |
| Apply first signal to the subject S | 530 |
| Measure current through and voltage induced across subject S | 540 |
| All frequencies complete | 550 |
| Determine measured impedance value(s) | 560 |
| Optionally determine one or more impedance parameter values | 570 |

No

Yes

## Fig. 5

30

**Fig. 6A**

**Fig. 6B**

| Determine first measured impedance value | 700 |

| Optionally determine impedance parameter value $R_0$ | 710 |

| Determine indicator value indicative of extracellular fluid levels | 720 |

| Display indicator to allow assessment of venous insufficiency | 730 |

| Determine if further analysis is required | 740 |

**Fig. 7**

```
┌──────────────────────┐
│   Determine first    │
│ indicator with subject│      800
│  in standing position │
└──────────────────────┘
            │
            ▼
┌──────────────────────┐
│   Treat subject for   │      810
│  venous insufficiency │
└──────────────────────┘
            │
            ▼
┌──────────────────────┐
│   Determine second   │
│  indicator value with │      820
│  subject in standing  │
│        position       │
└──────────────────────┘
            │
            ▼
┌──────────────────────┐
│  Determine difference │
│   between first and   │      830
│   second indicator    │
│        values         │
└──────────────────────┘
            │
            ▼
┌──────────────────────┐
│  Compare difference   │      840
│    to a reference     │
└──────────────────────┘
            │
            ▼
┌──────────────────────┐
│   Display indicator   │
│ indicative of results │      850
│    of comparison      │
└──────────────────────┘
```

# Fig. 8

**Fig. 9**

```
┌─────────────────┐
│   Determine first │
│ indicator value with │          1000
│  subject in supine │
│      position      │
└─────────────────┘
          │
          ▼
┌─────────────────┐
│  Position subject in │
│ standing position for │        1010
│ predetermined time │
│       period       │
└─────────────────┘
          │
          ▼
┌─────────────────┐
│  Determine second │
│ indicator value with │         1020
│  subject in supine │
│      position      │
└─────────────────┘
          │
          ▼
┌─────────────────┐
│ Determine difference │
│   between first and │           1030
│  second indicator │
│       values       │
└─────────────────┘
          │
          ▼
        No      ◇ Is difference ◇
        ◄───────◇ less than a    ◇         1040
                 ◇ reference?     ◇
                     │
                    Yes
                     │
                     ▼
┌─────────────────┐
│   Determine time │
│ taken for difference │          1050
│   to fall below │
│     reference    │
└─────────────────┘
          │
          ▼
┌─────────────────┐
│                 │
│  Display time taken │            1060
│                 │
└─────────────────┘
```

# Fig. 10

35

```
┌──────────────────────┐
│   Measure impedance   │      1100
│   values for body     │
│       segment         │
└──────────────────────┘
            │
            ▼
┌──────────────────────┐
│  Determine values of  │      1110
│  $R_0$, $R_\infty$ for the body │
│       segment         │
└──────────────────────┘
            │
            ▼
┌──────────────────────┐
│   Determine first     │      1120
│ indicator value using │
│ the parameter value $R_0$ │
└──────────────────────┘
            │
            ▼
┌──────────────────────┐
│   Determine second    │      1130
│ indicator value using │
│   an index-value $I$   │
└──────────────────────┘
            │
            ▼
┌──────────────────────┐
│ Compare first indicator│     1140
│    value to a first   │
│      reference        │
└──────────────────────┘
            │
            ▼
┌──────────────────────┐
│    Compare second     │      1150
│  indicator value to a │
│   second reference    │
└──────────────────────┘
            │
            ▼
┌──────────────────────┐
│   Display results of  │      1160
│      comparison       │
└──────────────────────┘
```

# Fig. 11

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 20060111652 A
- US 20050177062 A

- WO 0079255 A

### Non-patent literature cited in the description

- **NICOLAIDES AN.** Investigation of Chronic Venous Insufficiency: A Consensus Statement. *Circulation,* 2000, vol. 102, 126-163

- **MARIANNE FENECH ; MICHEL Y. JEFFRIN.** Extracellular and Intracellular Volume Variations During Postural Change Measured by Segmental and Wrist-ankle Bioimpedance Spectroscopy. *IEEE TRANSACTIONS ON BIOMEDICAL ENGINEERING,* 01 January 2004, vol. 51, 166-175